(19) Europäisches Patentamt
European Patent Office
Office européen des brevets

(11) **EP 3 689 303 B1**

(12) **FASCICULE DE BREVET EUROPEEN**

(45) Date de publication et mention
de la délivrance du brevet:
**04.08.2021  Bulletin 2021/31**

(51) Int Cl.:
***A61F 7/12*** *(2006.01)*    ***H01S 3/04*** *(2006.01)*

(21) Numéro de dépôt: **20153068.0**

(22) Date de dépôt: **22.01.2020**

(54) **DISPOSITIF DE REFROIDISSEMENT LOCALISÉ**

VORRICHTUNG ZUR LOKALISIERTEN KÜHLUNG

LOCALISED COOLING DEVICE

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **29.01.2019  FR 1900768**

(43) Date de publication de la demande:
**05.08.2020  Bulletin 2020/32**

(73) Titulaire: **Commissariat à l'énergie atomique
et aux énergies alternatives
75015 Paris (FR)**

(72) Inventeurs:
• **MERMILLOD-ANSLEME, Quentin
38054 GRENOBLE Cedex (FR)**
• **AUBERT, Nicolas
38054 GRENOBLE Cedex (FR)**
• **CHABROL, Claude
38054 GRENOBLE Cedex (FR)**
• **DUPOY, Mathieu
38054 GRENOBLE Cedex (FR)**

(74) Mandataire: **INNOV-GROUP
310, avenue Berthelot
69372 Lyon Cedex 08 (FR)**

(56) Documents cités:
EP-A2- 2 717 029    WO-A2-2007/104506
FR-A1- 3 055 796    JP-A- H11 153 754
US-B1- 6 378 321

**Description**

**Domaine technique de l'invention**

La présente invention se rapporte à un dispositif de refroidissement localisé, notamment d'un organe, tel que par exemple le cerveau humain.

**Etat de la technique**

Il est connu de traiter certaines maladies en effectuant un refroidissement localisé des tissus. C'est le cas par exemple des maladies comme l'épilepsie dans lesquelles un refroidissement localisé de la zone épileptogène permet de bloquer l'émergence de crises ou de limiter leur propagation. Ceci présente un intérêt pour les patients atteints de cette pathologie et que l'on ne peut traiter, du fait d'un caractère phamaco-résistant, par les médicaments classiques. Cependant, les dispositifs de refroidissement sont souvent peu adaptés à une utilisation en implantation chronique intra cérébrale. Certaines solutions connues, non adaptées à l'implantation, sont par exemple basées sur des refroidisseurs microfluidiques ou des refroidisseurs thermoélectriques utilisant l'effet Peltier.

Différentes solutions sont décrites dans les documents référencés US2007/005121A1, JP2006/015064A, US5620571A, US9362712B1 et WO2016/102351A1.

Par ailleurs, une autre technologie de refroidissement consiste à employer un cristal capable de refroidir lorsqu'il est excité par un faisceau laser.

Le cristal refroidissant est préférentiellement formé d'un matériau adapté pour fonctionner selon un principe dit de "fluorescence anti-Stokes". Ce principe consiste en une diffusion inélastique de la lumière, impliquant un échange d'énergie entre un photon incident de longueur d'onde déterminée et le réseau cristallin. La lumière diffusée par le cristal n'a ainsi pas la même longueur d'onde que la lumière incidente. Dans le cas d'un décalage anti-Stokes, la lumière diffusée présente une longueur d'onde plus faible que la lumière incidente et donc une énergie plus importante, ce qui aboutit au refroidissement du cristal. Des dispositifs utilisant ce principe de refroidissement ont notamment été décrits dans le brevet US6041610 et dans les demandes de brevet WO00/42683A1 et WO2018/051005A1.

Les solutions proposées dans les documents brevets référencés ci-dessus ne sont pas satisfaisantes, notamment d'un point de vue efficacité de refroidissement et d'un point de vue du rapport compacité/efficacité de refroidissement.

Le but de l'invention est de proposer un dispositif de refroidissement localisé présentant une efficacité accrue dans un design particulièrement compact.

**Exposé de l'invention**

Ce but est atteint par un dispositif de refroidissement localisé comprenant :

- Un organe de refroidissement,
- Un cristal à capacité de refroidissement par absorption d'un signal lumineux d'excitation proche infrarouge,
- Un système d'illumination destiné à fournir un signal lumineux d'excitation,
- Ledit cristal comportant une forme allongée autour d'un axe longitudinal entre une extrémité proximale et une extrémité distale et présentant une section transversale externe constante fermée et un canal central réalisé à partir de son extrémité distale, sur au moins une partie de sa longueur,
- Ledit organe de refroidissement comportant une tige encastrée par une première extrémité dans ledit canal central dudit cristal et comportant une deuxième extrémité saillante formant un doigt de refroidissement,
- Le système d'illumination étant configuré pour générer ledit signal lumineux d'excitation sous une forme annulaire.

Selon une particularité, le cristal présente une section transversale constante de forme annulaire entre son extrémité proximale et son extrémité distale.

Selon une autre particularité, le système d'illumination comporte une source de lumière configurée pour émettre un premier signal lumineux et un module de mise en forme dudit premier signal lumineux agencé entre ladite source de lumière et l'extrémité proximale dudit cristal et configuré pour générer ledit signal d'excitation sous la forme annulaire.

Selon une autre particularité, le module de mise en forme comporte une première lentille axicon et une deuxième lentille axicon.

Selon une autre particularité, le cristal est de type Yb:YLF ou Tm:Yb:YLF.

Selon une autre particularité, le doigt de l'organe de refroidissement présente une forme atraumatique.

De manière avantageuse, le dispositif comporte des moyens de recyclage de la fluorescence générée lors de l'excitation dudit cristal.

Selon une particularité, ledit cristal comporte une surface latérale et lesdits moyens de recyclage de la fluorescence comportent un ou plusieurs filtres dichroïques agencés en périphérie de ladite surface latérale du cristal, formant une enveloppe latérale dudit cristal qui est isolée thermiquement de la surface latérale du cristal.

De manière avantageuse, les moyens de recyclage de la fluorescence comportent des éléments fluorophores.

De manière avantageuse, ladite enveloppe comporte au moins deux filtres dichroïques et des éléments fluorophores sont agencés entre les deux filtres dichroïques.

Selon une particularité, ladite enveloppe est composée d'un cylindre monobloc ou de plusieurs plaquettes adjacentes assemblées entre elles de manière jointive.

Selon une particularité, le dispositif comporte des moyens de fixation de ladite enveloppe.

Selon une première réalisation particulière, les moyens de fixation comportent une rondelle portant ladite enveloppe et enfilée autour de la tige de l'organe de refroidissement.

Selon une particularité, la rondelle comporte une face située en vis-à-vis du cristal sur laquelle est déposé un revêtement en matériau réfléchissant.

Selon une deuxième réalisation particulière, lesdits moyens de fixation comportent une ou plusieurs entretoises annulaires positionnées autour de la surface latérale dudit cristal et supportant ladite enveloppe.

Selon une particularité, chaque entretoise comporte plusieurs lamelles souples venant en appui contre la surface latérale dudit cristal.

**Brève description des figures**

D'autres caractéristiques et avantages vont apparaître dans la description détaillée qui suit faite en regard des dessins annexés dans lesquels :

- Les figures 1A et 1B illustrent, vu en perspective, un principe de réalisation de la sonde du dispositif de refroidissement localisé conforme à l'invention ;
- La figure 2A représente, de manière schématique suivant une vue en coupe longitudinale, le dispositif de refroidissement localisé conforme à l'invention et illustre son principe de fonctionnement ;
- La figure 2B représente la sonde du dispositif de l'invention, vue suivant la coupe transversale A-A agrandie ;
- La figure 3 illustre le principe de réalisation du signal lumineux d'excitation employé dans le dispositif de l'invention ;
- La figure 4 représente plusieurs configurations possibles du doigt de refroidissement employé dans le dispositif de l'invention ;
- La figure 5A illustre, de manière schématique suivant une vue en coupe longitudinale axiale, un principe de réalisation de la sonde du dispositif de refroidissement localisé de l'invention ;
- La figure 5B représente la sonde du dispositif de l'invention, vue suivant la coupe transversale B-B agrandie et montre l'enveloppe de filtres dichroïques réalisée suivant une section circulaire ;
- La figure 5C représente la sonde du dispositif de l'invention, vue suivant la coupe transversale B-B agrandie et montre l'enveloppe de filtres dichroïques réalisée suivant une section octogonale ;
- La figure 6A représente, de manière schématique suivant une vue en perspective et en transparence, la sonde du dispositif de refroidissement localisé de l'invention, et illustre une première solution de fixation de l'enveloppe de filtres dichroïques ;
- La figure 6B représente la sonde du dispositif de l'invention de la figure 6A, vue suivant une coupe longitudinale axiale ;
- La figure 6C représente la sonde du dispositif de l'invention, vue suivant la coupe transversale C-C agrandie ;
- Les figures 6D et 6E représentent les deux faces de la rondelle de maintien de l'enveloppe de filtres, employée dans la sonde des figures 6A et 6B et la figure 6F illustre une réalisation particulière de la rondelle ;
- La figure 7A représente, de manière schématique suivant une vue en coupe longitudinale axiale, la sonde du dispositif de refroidissement localisé de l'invention, et illustre une deuxième solution de fixation de l'enveloppe de filtres dichroïques ;
- La figure 7B représente la sonde du dispositif de l'invention, vue suivant la coupe transversale D-D agrandie ;
- La figure 8 représente la structure de l'entretoise employée dans la sonde des figures 7A et 7B ;
- La figure 9 représente le spectre de la fluorescence émise par le cristal employé dans le dispositif de l'invention ;
- Les figures 10A et 10B illustrent le principe de recyclage de la fluorescence employé dans le dispositif de l'invention ;
- Les figures 11A et 11B représentent respectivement, en fonction de la longueur d'onde, un diagramme de densité optique et un diagramme d'absorption des deux filtres dichroïques sélectionnés pour un angle solide de 120°;
- Les figures 12A et 12B représentent respectivement, pour un dispositif avec ou sans filtres dichroïques, un diagramme montrant des spectres de fluorescence normalisés à la température T = 293K et un diagramme de rendement de refroidissement en fonction de la longueur d'onde ;
- Les figures 13A et 13B représentent respectivement, pour un dispositif avec ou sans filtres dichroïques, un diagramme de dépendance en température du rendement de refroidissement et un diagramme de dépendance en température de la puissance de refroidissement, pour une excitation à 1020nm et une puissance laser de 19W ;

**Description détaillée d'au moins un mode de réalisation**

L'invention concerne un dispositif de refroidissement localisé, en particulier d'un organe, en vue de refroidir ses tissus. Ledit organe sera par exemple le cerveau humain.

De manière non limitative, le dispositif de l'invention sera par exemple adapté au traitement de différentes pathologies, par exemple :

- Crise d'épilepsie,
- Traumatisme crânio-encéphalique,
- Neuro-Cancer,
- Maladie de Parkinson ou autres maladies du mouvement telles que les dystonies, les tremblements essentiels, la chorée de huntington.

Bien entendu, moyennant certaines adaptations, il faut comprendre qu'il pourra être employé pour traiter d'autres pathologies.

Dans le cas du traitement d'une crise d'épilepsie, la source de froid générée est destinée à être mise en contact des foyers épileptogènes ou de toute autre zone du cerveau 30 accessible chirurgicalement en utilisant des méthodes mini invasives stéréotaxiques au travers d'une perforation ("drill") crânienne de quelques millimètres. Le refroidissement généré contribue alors à l'arrêt de la crise ou à bloquer son émergence.

D'autres applications pourront être envisagées, notamment dans le domaine spatial pour de l'imagerie infrarouge refroidie, ou dans le domaine de la spectrométrie refroidie.

Pour une application médicale, le dispositif de refroidissement 1 localisé de l'invention comporte une sonde 10 biocompatible et implantable de manière à pouvoir agir sur commande à tout instant, par exemple lorsque l'apparition d'une crise d'épilepsie est détectée, en utilisant des algorithmes de détection des crises en boucle fermée ("closed loop").

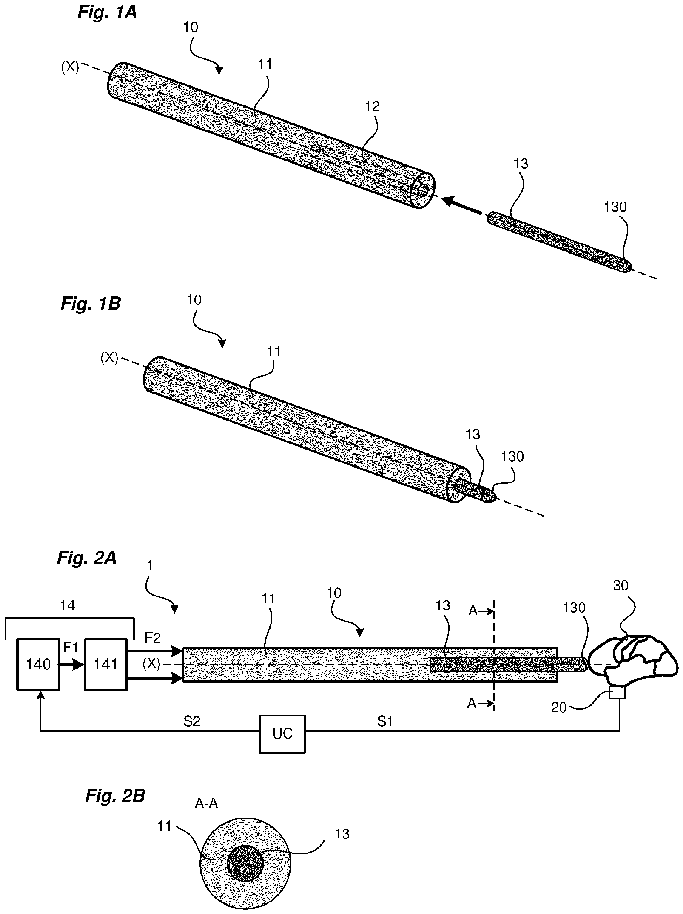

Selon l'application visée, des moyens de détection 20, ne faisant pas l'objet de la présente demande, sont par exemple employés pour détecter l'apparition de la pathologie à traiter. Dans le cas d'une crise d'épilepsie, ces moyens de détection 20 sont implantés au niveau crânien (par exemple cerveau 30 sur la figure 2A) pour détecter une zone épileptogène. Lorsque l'apparition d'une crise est détectée, les moyens de détection 20 envoient un signal S1 à une unité de commande et de traitement UC. L'unité de commande et de traitement UC émet alors une commande (S2) de réponse adaptée vers la sonde 10 du dispositif. Le dispositif de refroidissement de l'invention est commandé pour générer un refroidissement adapté à la pathologie traitée. S'il s'agit d'une crise d'épilepsie, l'intensité du refroidissement et sa durée d'application seront préférentiellement liés au niveau d'intensité de la crise qui a été mesuré par les moyens de détection 20. Pour générer une commande adaptée de la sonde du dispositif de refroidissement, l'unité de commande et de traitement UC comporte préférentiellement un module d'analyse destiné à analyser le signal S1 reçu en provenance des moyens de détection et à déterminer un traitement adapté.

L'unité de commande et de traitement UC fait avantageusement partie du dispositif de refroidissement 1 de l'invention. Elle comporte au moins un microprocesseur et des moyens de mémorisation. Elle est destinée à exécuter des instructions logicielles représentatives d'une séquence de traitement de la pathologie par le dispositif. Elle comporte notamment des moyens de commande d'un système d'illumination 14 qui sera détaillé ci-dessous. Elle comporte également une ou plusieurs interfaces de communication destinées à communiquer avec les différentes entités, notamment les moyens de détection décrits ci-dessus. Les liaisons de communication pourront être filaire ou sans-fil.

La sonde 10 du dispositif de refroidissement 1 de l'invention comporte un cristal 11 refroidissant. Le cristal 11 refroidissant est préférentiellement formé d'un matériau adapté pour fonctionner selon un principe dit de "fluorescence anti-Stokes". Ce principe consiste en une diffusion inélastique de la lumière, impliquant un échange d'énergie entre un photon incident de longueur d'onde déterminée et le réseau cristallin. La lumière diffusée par le cristal n'a ainsi pas la même longueur d'onde que la lumière incidente. Dans le cas d'un décalage anti-Stokes, la lumière diffusée présente une longueur d'onde plus faible que la lumière incidente mais avec une énergie plus importante, ce qui aboutit au refroidissement du cristal.

De manière non limitative, le cristal employé sera préférentiellement formé de toute matrice hôte transparente dans le proche infrarouge (900-1100nm), présentant des phonons de basse énergie. Cette matrice sera dopée préférentiellement en ions ytterbium ($Yb^{3+}$). Il s'agira par exemple d'un cristal de type 10% wt. Yb: YLF. Sa longueur d'onde d'excitation est comprise entre 1010 nm et 1040 nm environ, préférentiellement 1020 nm.

Bien entendu, toute autre composition de cristal pourra être envisagée comme par exemple celle d'un cristal de YLF co-dopé 5% Yb - 0.0016% Tm. D'une manière générale, tous les ions de type lanthanides pourront être envisagés. Une attention particulière sera apportée à la pureté des cristaux utilisés. On utilisera par exemple des composants de pureté 5N lors de la fabrication de la matrice hôte.

Dans le cas d'un cristal dopé avec de l'ytterbium, le schéma de refroidissement est le suivant et a déjà été décrit dans la demande de brevet WO2018/051005A1 et illustré dans la demande de brevet US2017/0137684A1 : soit un électron initialement sur le niveau E4. Cet électron vient être porté à un état excité E5 par l'absorption d'un photon incident de longueur d'onde 1020 nm. Il est ensuite porté dans les états d'énergies supérieurs (E6, E7) par l'absorption de phonons acoustiques du réseau cristallin. Cet électron se désexcite de manière radiative vers les niveaux d'énergies fondamentaux (E1-E4) en émettant un photon de longueur d'onde de fluorescence moyenne (environ 990 nm). Un phonon est encore absorbé de manière à ce que l'électron revienne à son état d'équilibre.

Selon une particularité de l'invention, en référence aux figures 1A et 1B ainsi qu'aux figures 2A et 2B, le cristal 11 est réalisé suivant une forme allongée selon un axe longitudinal (X), entre une extrémité proximale recevant en premier un signal lumineux d'excitation et une extrémité distale pouvant être située au plus près de l'organe 30 à traiter. Le cristal 11 présente une section transversale externe constante fermée et suivant son axe longitudinal un espace central creux formant un canal 12 réalisé sur toute ou partie de sa longueur et définissant ainsi une surface interne cylindrique d'échange thermique.

De manière avantageuse, la section transversale du cristal 11 est ainsi de forme annulaire au moins sur la portion de longueur incluant le canal central 12.

La sonde comporte également un organe de refroidissement 13 présentant une surface externe en contact mécanique avec la surface interne du cristal. L'organe de refroidissement 13 est destiné à capter la variation de température négative générée par le cristal refroidissant lorsque ce dernier est excité de manière adaptée.

Selon une particularité, cet organe de refroidissement 13 comporte une structure allongée entre une première extrémité et une deuxième extrémité. Il comporte une tige cylindrique de révolution venant s'encastrer par sa première extrémité dans ledit canal central 12 du cristal 11 et un doigt 130 de refroidissement (appelé également "doigt froid") de forme atraumatique réalisé à la deuxième extrémité de ladite tige. L'organe de refroidissement 13 n'est avantageusement maintenu que par l'unique liaison mécanique d'encastrement dans le canal central 12 du cristal 11.

L'organe de refroidissement 13 est avantageusement réalisé en saphir, ce matériau présentant une conductivité thermique élevée, un niveau de transparence adapté dans le proche infrarouge et des caractéristiques de biocompatibilité.

La tige de l'organe de refroidissement peut être insérée axialement dans le canal sur toute la longueur du canal jusqu'à butée lorsque le canal est borgne, de sorte que seul le doigt de refroidissement 130 reste saillant par rapport à l'extrémité distale du cristal 11.

Lorsque l'organe de refroidissement 13 est encastré, la surface latérale externe de sa tige est en contact mécanique avec la surface interne du cristal 11, permettant un échange thermique entre les deux éléments.

Le doigt 130 de refroidissement est destiné à venir en contact avec l'organe à refroidir (cerveau 30 sur la figure 2A).

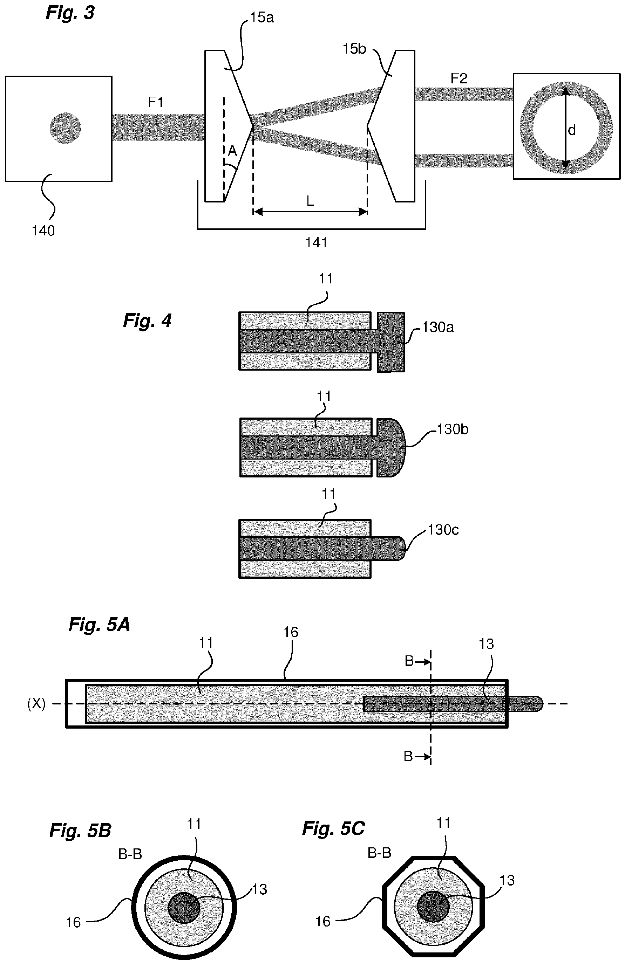

En référence à la figure 4, à son extrémité, le doigt 130 peut par exemple présenter une forme évasée formant un T (130a) ou un champignon (130b), ou une forme droite (130c) en bâtonnet se terminant par un dôme arrondi. D'autres formes de type atraumatique pourraient être envisagées.

Selon une particularité de l'invention, le dispositif 1 comporte un système d'illumination 14 comprenant au moins une source 140 destinée à générer un signal lumineux. Le signal est employé pour exciter le cristal selon le principe décrit ci-dessus.

Le système d'illumination est configuré pour générer un signal d'excitation ayant une section transversale en anneau pour n'exciter que le cristal, cette section étant avantageusement identique ou au moins inscrite dans la section transversale du cristal 11 de manière à laisser non-excité le canal central 12 du cristal 11 où se situe l'organe de refroidissement 13. Le système d'illumination comporte ainsi des moyens de génération de ce signal d'excitation à section transversale annulaire.

De manière non limitative, le signal d'excitation peut ainsi être obtenu grâce à une source lumineuse délivrant nativement un signal d'excitation de section annulaire.

En variante, la source lumineuse 140 est avantageusement un laser à faisceau gaussien. Dans ce cas, il s'avère que l'encastrement de l'organe de refroidissement 13 dans le cristal 11 ne permet pas une excitation gaussienne du cristal car l'intégralité de la puissance laser serait alors guidée axialement vers l'organe de refroidissement 13 jusqu'à la zone à refroidir. Selon une particularité, le système d'illumination 14 du dispositif comporte ainsi un module 141 de mise en forme du faisceau gaussien F1 émis par le laser en un faisceau F2 à section annulaire constante permettant ainsi de n'exciter que le cristal 11. Le faisceau F2 à section annulaire qui est généré présente avantageusement une section transversale identique ou au moins inscrite dans la section transversale du cristal 11 de manière à laisser non-excité le canal central 12 du cristal 11 où se situe l'organe de refroidissement 13, et à concentrer la puissance du laser sur le cristal 11 situé en périphérie.

Comme représenté sur la figure 3 et de manière non limitative, le module 141 de mise en forme du faisceau gaussien du laser peut comporter deux axicons 15a, 15b en série, tête bêche et dont les sommets sont séparés d'une distance L non nulle. Chaque axicon peut être formé d'une lentille de forme conique dont l'angle A sera choisi de manière adaptée. L'utilisation d'un doublet d'axicons présente un intérêt dans sa facilité de mise en œuvre et dans sa résistance au flux. Le diamètre de l'anneau obtenu en sortie du module de mise en forme vaut d = 2xLxtan(A(n-1)) et s'avère facilement réglable en jouant sur la distance L entre les sommets des deux lentilles et sur l'angle A de chaque lentille.

Bien entendu, le module 141 de mise en forme pourrait être réalisé autrement. A titre d'exemple, les axicons pourraient être remplacés par une lame à retard de phase.

De manière avantageuse, pour améliorer l'efficacité du dispositif et de sa sonde, il est possible d'employer des moyens de recyclage de la fluorescence générée par l'excitation du cristal 11 par le signal lumineux.

On verra ci-dessous que ces moyens de recyclage intégrés à la sonde sont avantageusement formés d'un ou plusieurs filtres 16 dichroïques agencés autour du cristal 11 sous la forme d'une enveloppe latérale et permettent de recycler les photons de fluorescence de basse énergie (par basse énergie, on entend inférieur à l'énergie de fluorescence moyenne) en les réfléchissant vers le cristal 11. Grâce à ces moyens, 50% de la fluorescence est réabsorbée par le cristal 11, ce qui permet de doubler le rendement de refroidissement et de diviser par deux la fluorescence délétère du cristal. Par ailleurs, on augmente également la vitesse de refroidissement du cristal 11.

Le principe de l'invention réside dans le recyclage des photons de fluorescence d'énergies inférieures à l'énergie de fluorescence moyenne définie telle que :

$$h\nu_f = \frac{hc}{\lambda_f}$$

Où:

- h représente la constante de Planck,
- vf représente la fréquence moyenne des photons de fluorescence,
- c représente la vitesse de la lumière dans le vide,

$\lambda$f représente pour sa part la longueur d'onde moyenne des photons de fluorescence telle que :

$$\lambda_f = \frac{\int \lambda S(\lambda)d\lambda}{\int S(\lambda)d\lambda}$$

Où S représente une quantité mesurée lors de l'expérience correspondant à une densité spectrale d'émission (nombre de photons émis par intervalle de longueur d'onde).

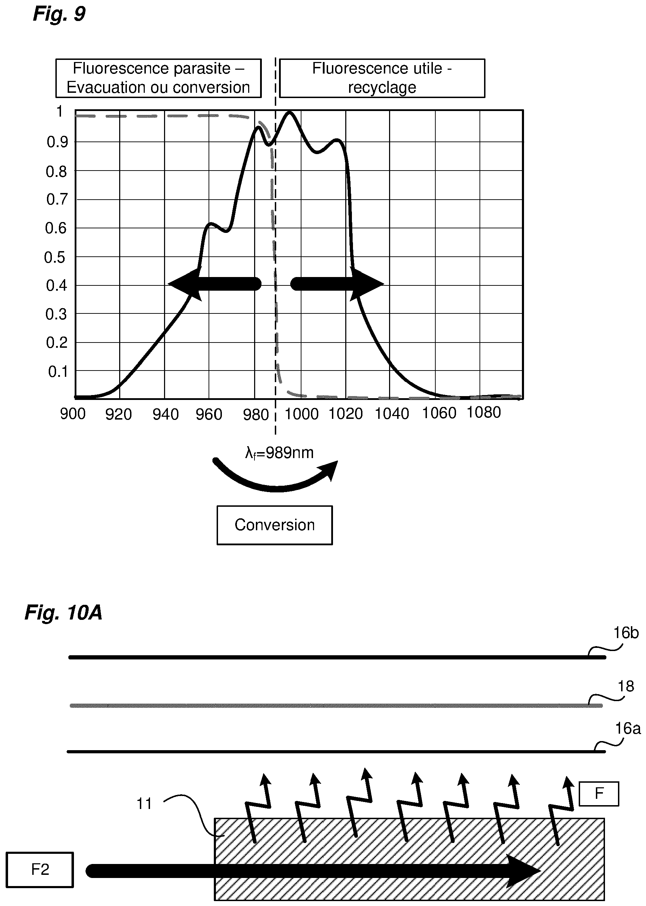

La Figure 9 illustre un spectre de fluorescence normalisé pour un cristal 10% wt. Yb$^{3+}$ :YLF. Pour ce cristal, la longueur d'onde de fluorescence moyenne mesurée est $\lambda$f= 989 nm. Cette longueur d'onde correspond à un rendement de refroidissement ($\eta_c$) nul, lequel est défini par l'équation suivante :

$$\eta_c(\lambda, T) = \frac{P_{cool}}{P_{abs}} = 1 - \eta_{ext}\left(\frac{\alpha_r(\lambda, T)}{\alpha_r(\lambda, T) + \alpha_b}\right)\frac{\lambda}{\lambda_f(T)}$$

Où $\eta_{ext}$ représente le rendement quantique externe, $\alpha_r(\lambda, T)$ représente l'absorption résonante dépendante de la longueur d'onde d'excitation et de la température et $\alpha_b$ l'absorption résiduelle causée par la présence d'impuretés dans le cristal.

Le principe consiste ainsi à utiliser un filtre dichroïque 16 sélectif en longueur d'onde (filtre idéal illustré par les pointillés sur la figure 9), lequel dans le cas idéal permet de réfléchir les photons de fluorescence de longueurs d'ondes supérieures à $\lambda$f qui pourront de nouveau être réabsorbés par le cristal 11 et ainsi d'augmenter le rendement de refroidissement. Les longueurs d'ondes transmises par les filtres 16 sont celles dont les longueurs d'onde sont inférieures à $\lambda$f.

Comme représenté sur les figures 5A à 5C, les filtres dichroïques 16 du dispositif 1 sont assemblés sous la forme d'une enveloppe latérale agencée autour de la surface latérale du cristal 11, de manière coaxiale par rapport à celle-ci, et ne venant pas en contact avec celle-ci. L'enveloppe peut être réalisée sous la forme d'un tube cylindrique monobloc ou de plusieurs plaquettes assemblées entre elles de manière jointive. Dans ce dernier cas, chaque plaquette peut être plane ou incurvée. L'ensemble des filtres assemblés forme alors un cylindre traversant de forme prismatique (par exemple à section externe circulaire sur la figure 5B ou à section externe octogonale sur la figure 5C) d'axe confondu avec l'axe (X).

Pour fixer les filtres autour du cristal, les maintenir en position et former ainsi ladite enveloppe, différents moyens de fixation peuvent être envisagés.

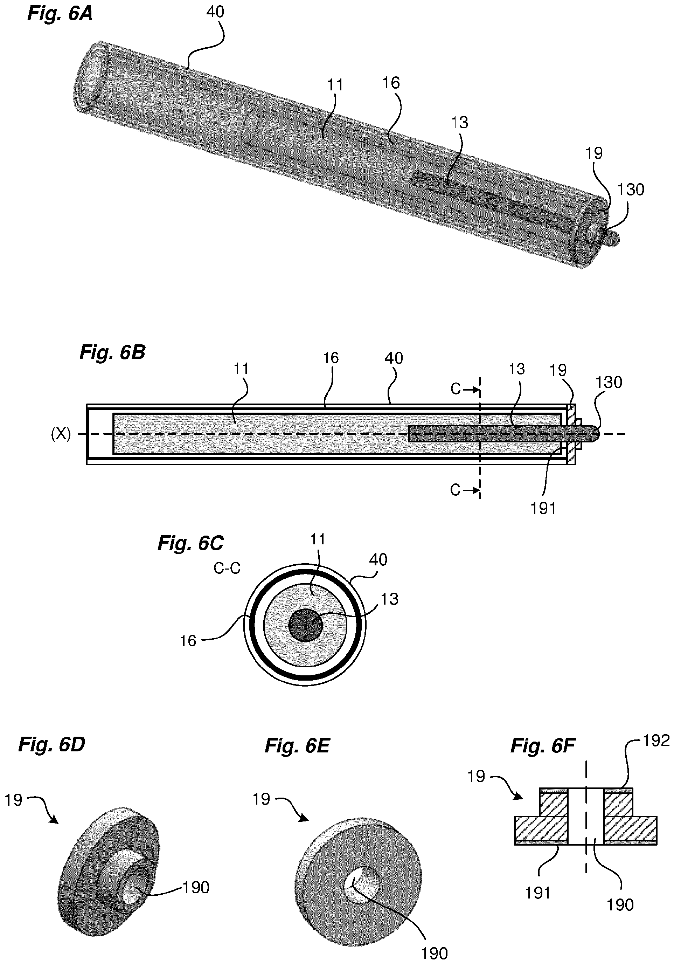

En référence aux figures 6A à 6E, une première solution de fixation des filtres 16, particulièrement avantageuse, consiste à munir l'enveloppe cylindrique du dispositif d'une rondelle 19 ou anneau de fixation venant obturer l'enveloppe à l'une de ses extrémités, formant ainsi l'une de ses bases. La rondelle 19 présente une ouverture axiale 190 de diamètre adapté pour être enfilé de manière ajustée autour de la tige de l'organe de refroidissement 13. La rondelle 19 est enfilée du côté du doigt de refroidissement 130. Elle est positionnée à une distance non nulle du cristal 11 et ne vient donc pas en butée contre celui-ci de manière à éviter tout échauffement parasite en fonctionnement. La rondelle 19 porte les filtres dichroïques 16 et les maintient uniquement par sa liaison mécanique avec la tige de l'organe de refroidissement 13.

De manière non limitative, la rondelle 19 est réalisée dans un matériau isolant thermiquement tel que le verre, le silicium ou un matériau obtenu par un procédé de type sol-gel.

Comme représenté sur les figures 6D à 6F, de manière non limitative, la rondelle 19 peut être réalisée sous la forme d'une pièce comprenant deux sections cylindriques de diamètres différents, formant entre elles un épaulement.

De manière avantageuse, comme représenté sur la figure 6F, sur sa face située en vis-à-vis du cristal 11, la rondelle peut porter un revêtement 191 réfléchissant, réalisé par exemple en or ou en argent.

De manière avantageuse encore, sur la face opposée, c'est-à-dire sur la face située du côté du doigt froid, la rondelle peut porter un revêtement 192 en silice ($SiO_2$ - Figure 6F), destiné à faire barrière thermique et à mieux isoler thermiquement le doigt de refroidissement 130. La rondelle 19 peut porter l'un ou l'autre de ces deux revêtements.

Cette première solution de fixation des filtres présente notamment les avantages suivants :

- Elle permet d'éviter tout échauffement parasite et des pertes par conduction thermique, car ni l'enveloppe de filtres 16, ni la rondelle 19 ne viennent au contact du cristal 11; Dans cette configuration, seul l'organe de refroidissement 13 est au contact du cristal 11 ;

- Elle permet de relâcher les contraintes sur le choix du matériau pour le support de fixation (autrement dit, il ne sera pas forcément nécessaire d'employer une rondelle 19 réalisée dans un matériau transparent dans le proche infrarouge) ;

- La rondelle 19 est facile à fabriquer, par exemple par usinage ;

Sur les figures 6A à 6C, la sonde comporte également une enveloppe d'encapsulation 40 venant se positionner autour de l'enveloppe de filtres. Cette enveloppe d'encapsulation 40 peut être employée dans tous les modes de réalisation décrits dans la présente demande.

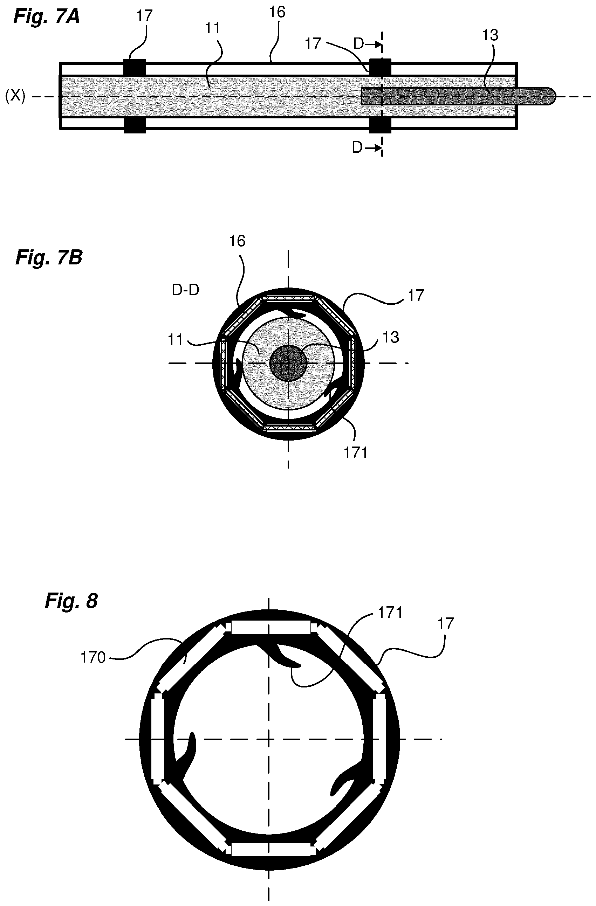

Une autre solution de fixation représentée sur les figures 7A et 7B consiste à doter le dispositif d'une ou plusieurs entretoises 17 enfilées autour du cylindre formé par le cristal 11. Les figures 7A et 7B illustrent le principe d'installation de ces entretoises (par exemple au nombre de deux) et la figure 8 représente une entretoise de manière plus spécifique. Les entretoises devront être réalisées dans un matériau transparent dans le proche infrarouge (essentiellement 900-1100 nm), et présentant une conductivité thermique faible, afin d'éviter tout échauffement parasite et pertes pas conduction thermique en fonctionnement.

Les entretoises 17 ont ainsi pour fonction de :

- Positionner les filtres dichroïques 16 employés et maintenir le cristal 11 et chaque filtre 16 centré l'un par rapport à l'autre. Chaque entretoise 17 est pourvue de plusieurs ouvertures 170 sur son pourtour pour y glisser dans chacune un filtre 16 distinct (réalisé par exemple sous la forme d'une plaquette plane ou incurvée).

- Permettre d'absorber les contraintes thermomécaniques (variation de températures des différents éléments et éventuels chocs) :
  ∘ Ces entretoises 17 sont pourvues de lamelles 171 souples (la forme n'est pas limitative) dont le but est :

    ▪ D'isoler thermiquement le cristal des parois des filtres par la limitation des interfaces cristal/entretoises et entretoises/filtres,

    ▪ De permettre un jeu mécanique de quelques $\mu$m à quelques centaines de $\mu$m, pour faciliter l'assemblage tout en maintenant le cristal 11 centré par rapport aux filtres. Ces lamelles 171 sont suffisamment souples pour maintenir le cristal tout en exerçant une légère pression sur ce dernier. L'état de surface de ces lamelles 171 devra être le plus "propre" possible afin de ne pas endommager le cristal 11 lors du processus d'assemblage,

■ Afin d'anticiper les futurs passages de tests de résistance aux chocs pour le passage du processus normatif, ces lamelles 171 seront suffisamment élastiques pour se déformer légèrement de manière à résister aux variations thermomécaniques dues aux changements de température ou aux chocs. Nous positionnons par exemple deux entretoises 17 afin d'assurer un maintien mais également de limiter des phénomènes de résonance si nous plaçons ces entretoises de manière asymétrique.

Par ailleurs, il faut noter que l'un des critères les plus importants pour la faisabilité d'une sonde implantable dans le cerveau est de limiter l'échauffement des tissus à 1°C (38°C absolu). Des simulations numériques ainsi que des mesures expérimentales ont ainsi été réalisées pour quantifier l'impact de la fluorescence sur un volume d'eau à 37°C. Les simulations et les expériences ont montré un échauffement délétère très supérieur à 38°C à proximité du cristal 11 dans les temps caractéristiques pour obtenir un volume refroidi inférieur à 27°C de l'ordre du mm$^3$. Il s'avère alors que l'utilisation des filtres dichroïques permet de diminuer d'environ 45% cet échauffement parasite, et qu'elle permet également de doubler le volume refroidi inférieur à 27°C. Par ailleurs, les filtres dichroïques 16 permettent d'augmenter la dynamique de refroidissement et d'atteindre un volume refroidi de l'ordre du mm$^3$ en moins de 10 secondes contre 30 secondes pour une sonde sans filtre. Ce dernier point est un autre critère important dans la faisabilité d'une sonde implantable de refroidissement de foyer épileptogène. En effet, plus le volume refroidi est atteint rapidement, moins le foyer épileptogène a le temps de s'étendre et donc plus le dispositif est efficace dans le traitement de la crise épileptique.

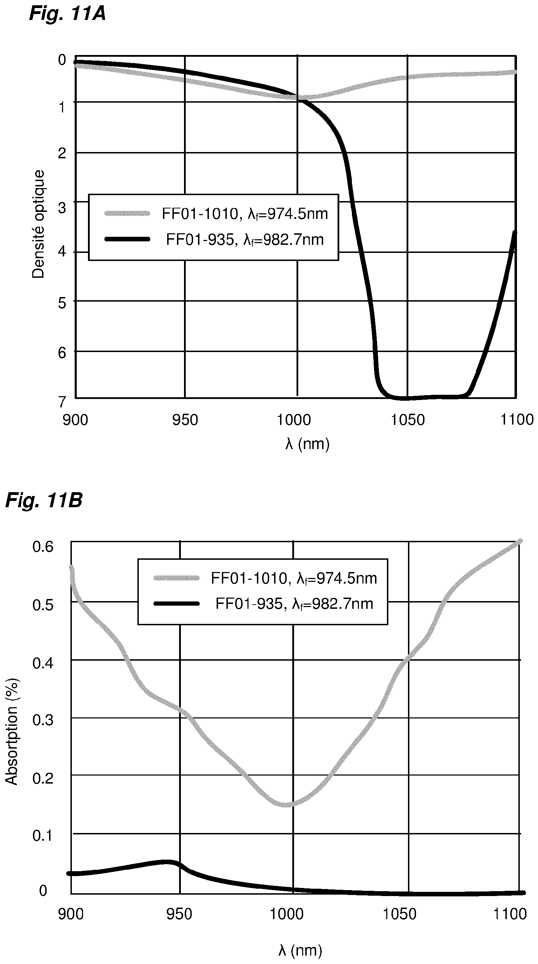

Dans le cadre de cette invention, cinq filtres dichroïques de la marque "Semrock" ont été testés et caractérisés expérimentalement. Deux filtres, référencés FF01-935 et FF01-1010, ont été sélectionnés pour leurs fortes réflectivités, même pour les grands angles d'incidences (figure 11A). Le filtre FF01-935 se distingue tout particulièrement avec une densité optique supérieure à 7 (limite de détection) aux longueurs d'ondes d'intérêt. Par ailleurs, l'absorption de ces filtres aux longueurs d'ondes de fluorescence du cristal reste inférieure à 1% (figure 11B). L'échauffement parasite des filtres est donc négligeable, notamment pour le filtre FF01-935 dont l'absorption est inférieure à 0.05%.

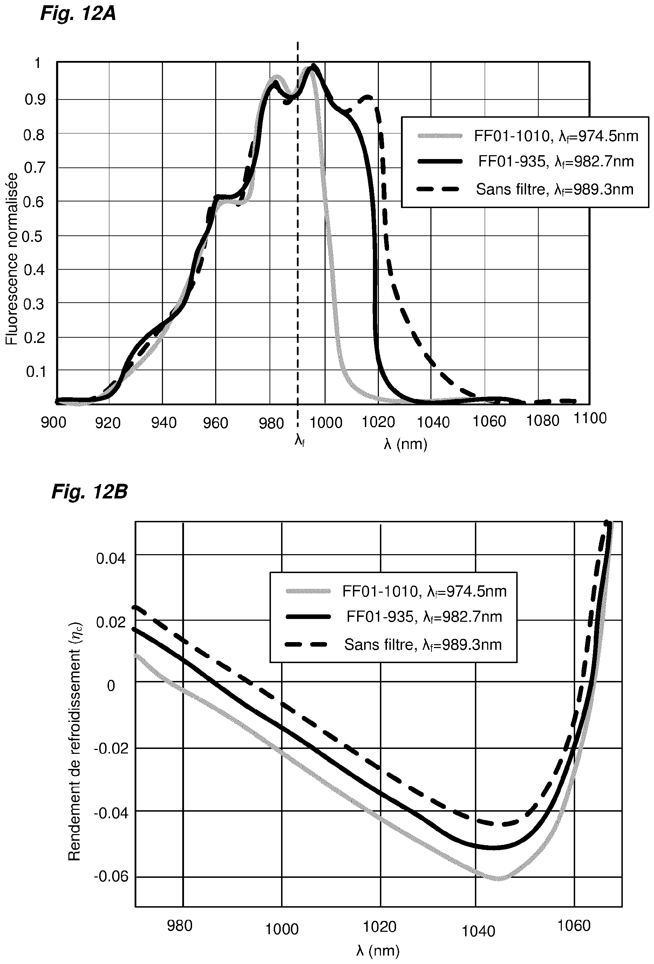

Une caractérisation spectrale de ces filtres a été réalisée sur un banc optique en positionnant les filtres à environ un millimètre de la surface du cristal et en mesurant les spectres de fluorescence transmis. Cette caractérisation a permis de vérifier que les longueurs d'ondes de coupures des filtres sont au-delà de λf (figure 12A). Le filtre FF01 - 1010 a la longueur d'onde de coupure la plus proche de λf, ce qui se traduit par un gain de 40% sur le rendement de refroidissement contre 20% pour le filtre FF01-935 (figure 12B).

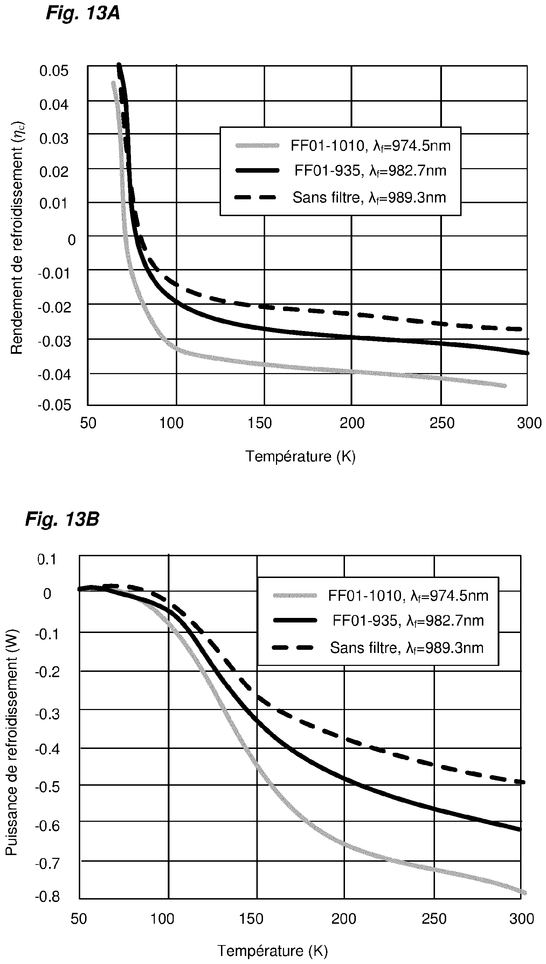

Le rendement de refroidissement du cristal dépend également de la température (Voir équation du rendement de refroidissement $\eta_c$ ci-dessus). Ce dernier diminue avec la température et devient positif à partir d'une certaine température correspondant à la température globale minimale accessible (désigné gMAT) du cristal (Figures 13A et 13B). On remarque que l'utilisation de filtres dichroïques 16 permet de diminuer de quelques degrés cette température gMAT du cristal et d'atteindre la température de l'azote liquide (77 K).

Par ailleurs, selon une particularité du dispositif de l'invention, il est également possible de convertir les longueurs d'ondes qui sont transmises par les filtres dichroïques 16 en longueurs d'ondes utiles pour le refroidissement. En effet les longueurs d'ondes parasites sont d'énergies plus élevées que les longueurs d'ondes utiles. Il est donc aisé, par phénomène de fluorescence, de diminuer l'énergie de la fluorescence parasite pour ensuite l'exploiter à nouveau.

La figure 9 montre le spectre de la fluorescence du cristal 11. La partie de droite du spectre est recyclée par les filtres dichroïques 16, tandis que la partie de gauche doit être évacuée ou recyclée afin de favoriser le refroidissement du cristal. Une méthode pour convertir cette lumière parasite de haute énergie en lumière de plus faible d'énergie est d'utiliser des éléments fluorophores 18 dont le spectre d'excitation sera centré autour de 970nm et dont le spectre d'émission sera compris entre 1000nm et 1060nm. Ce type d'éléments fluorophores 18 est notamment employé dans des applications d'imagerie médical. En effet cette gamme de longueur d'onde correspond à une fenêtre de transparence des tissus biologiques.

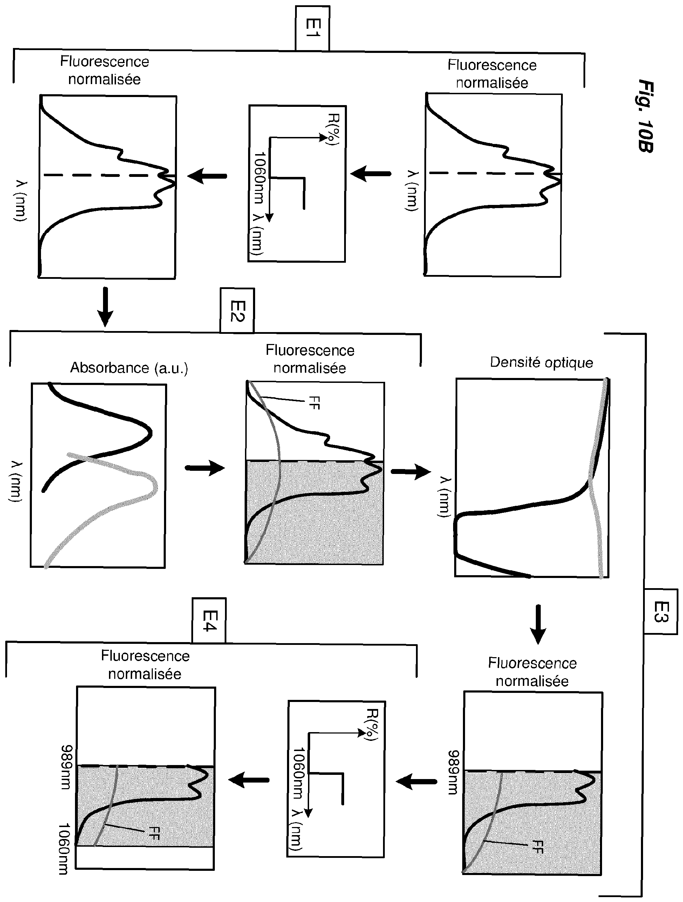

A titre d'exemple, les figures 10A et 10B illustrent ainsi le principe global de recyclage de la fluorescence pouvant être employé dans le dispositif de l'invention :

Etape E1 : La fluorescence F émise par le cristal 11 lors de l'excitation par le laser (faisceau F2) passe à travers un premier filtre dichroïque 16a. A ce stade, le premier filtre dichroïque 16a n'a aucun effet sur le spectre.

Etape E2 : La fluorescence émise rejoint alors les éléments fluorophores 18. Ceux-ci absorbent la fluorescence aux longueurs d'ondes inférieures à environ 990 nm et réémettent de la fluorescence à des longueurs d'ondes supérieures à environ 990 nm (partie grise du diagramme). La courbe référencée FF correspond à la courbe de fluorescence des éléments fluorophores.

Etape E3 : La fluorescence réémise traverse le deuxième filtre dichroïque 16b. Ce filtre réfléchit alors la fluorescence réémise par les éléments fluorophores 18 aux longueurs d'ondes supérieures à 990 nm (partie grise du diagramme).

Etape E4 : La fluorescence réfléchie par le deuxième filtre dichroïque 16b traverse à nouveau le premier filtre dichroïque 16a (les éléments fluorophores situées entre les deux filtres sont alors sans effet). Le premier filtre dichroïque 16a sélectionne alors les longueurs d'ondes à réinjecter dans le cristal pour recyclage. Sur la figure 10B, il s'agit des longueurs d'ondes comprises entre 990 nm et 1060 nm (partie grise du diagramme). En effet, pour des longueurs d'ondes qui vont au-delà de 1060 nm, le cristal 11 peut commencer à chauffer, ce qui aurait l'effet inverse de celui recherché.

Grâce à cette architecture optique, la lumière revenant sur le cristal est constituée uniquement de lumière utile pour le refroidissement optique.

Dans cette architecture optique, les deux filtres dichroïques 16a, 16b et les éléments fluorophores 18 pourront être assemblés dans une même plaquette destinée à être agencée autour du cristal selon les modes de réalisation décrits ci-dessus en liaison avec les figures 5A à 8.

La solution de l'invention présente de nombreux avantages, parmi lesquels :

- Présence d'une grande surface de contact entre l'organe de refroidissement et le cristal, permettant d'augmenter les échanges thermiques ;

- Solution qui permet d'éviter l'emploi de colles pour fixer l'organe de refroidissement sur le cristal, éliminant les risques d'échauffement parasites et améliorant l'échange thermique entre les deux éléments ;

- Solution particulièrement résistante, même lors de l'application des cycles thermiques de fonctionnement ;

- Solution qui permet d'employer un doigt de refroidissement de petite taille, limitant ainsi les pertes thermiques avec l'extérieur et permettant de mieux localiser le refroidissement effectué ;

- Une solution présentant un rendement élevé, notamment grâce à l'emploi des moyens de recyclage de la fluorescence (filtres dichroïques+éléments fluorophores) ;

- Une solution mécanique fiable et simple à fabriquer, grâce notamment à l'emploi de la rondelle de fixation ou des entretoises et au fait de limiter l'emploi de colles pour assembler les éléments entre eux ; On peut noter que l'enveloppe de filtres dichroïques 16 peut être maintenue uniquement par l'organe de refroidissement, grâce à la rondelle 19, évitant tout contact entre la rondelle et le cristal, permettant ainsi d'éviter tout échauffement parasite en fonctionnement ;

## Revendications

1. Dispositif de refroidissement localisé comprenant :

   - Un organe de refroidissement (13),
   - Un cristal (11) à capacité de refroidissement par absorption d'un signal lumineux d'excitation proche infrarouge,
   - Un système d'illumination (14) destiné à fournir un signal lumineux d'excitation:
   - Ledit cristal (11) comportant une forme allongée autour d'un axe longitudinal entre une extrémité proximale et une extrémité distale et présentant une section transversale externe constante fermée et un canal central réalisé à partir de son extrémité distale, sur au moins une partie de sa longueur,
   - Ledit organe de refroidissement (13) comportant une tige encastrée par une première extrémité dans ledit canal central dudit cristal et comportant une deuxième extrémité saillante formant un doigt de refroidissement (130)
   - Le système d'illumination étant configuré pour générer ledit signal lumineux d'excitation sous une forme annulaire.

2. Dispositif selon la revendication 1, **caractérisé en ce que** le cristal (11) présente une section transversale constante de forme annulaire entre son extrémité proximale et son extrémité distale.

3. Dispositif selon la revendication 1, **caractérisé en ce que** le système d'illumination comporte une source de lumière configurée pour émettre un premier signal lumineux et un module (141) de mise en forme dudit premier signal lumineux agencé entre ladite source (140) de lumière et l'extrémité proximale dudit cristal (11) et configuré pour générer ledit signal d'excitation sous la forme annulaire.

4. Dispositif selon la revendication 3, **caractérisé en ce que** le module (141) de mise en forme comporte une première lentille axicon (15a) et une deuxième lentille axicon (15b).

5. Dispositif selon l'une des revendications 1 à 4, **caractérisé en ce que** le cristal (11) est de type Yb:YLF ou Tm:Yb:YLF.

6. Dispositif selon l'une des revendications 1 à 5, **caractérisé en ce que** le doigt (130) de l'organe de refroidissement (13) présente une forme atraumatique.

7. Dispositif selon l'une des revendications 1 à 6, **caractérisé en ce qu'**il comporte des moyens de recyclage de la fluorescence générée lors de l'excitation dudit cristal (11).

8. Dispositif selon la revendication 7, **caractérisé en ce que** ledit cristal (11) comporte une surface latérale et **en ce que** lesdits moyens de recyclage de la fluorescence comportent un ou plusieurs filtres dichroïques (16) agencés en périphérie de ladite surface latérale du cristal, formant une enveloppe latérale dudit cristal qui est isolée thermiquement de la surface latérale du cristal.

9. Dispositif selon la revendication 8, **caractérisé en ce que** les moyens de recyclage de la fluorescence comportent des éléments fluorophores (18).

10. Dispositif selon la revendication 9, **caractérisé en ce que** ladite enveloppe comporte au moins deux filtres dichroïques (16a, 16b) et **en ce que** des éléments fluorophores (18) sont agencés entre les deux filtres dichroïques.

11. Dispositif selon la revendication 9 ou 10, **caractérisé en ce que** ladite enveloppe est composée d'un cylindre monobloc ou de plusieurs plaquettes adjacentes assemblées entre elles de manière jointive.

12. Dispositif selon l'une des revendications 9 à 11, **caractérisé en ce qu'**il comporte des moyens de fixation de ladite enveloppe.

13. Dispositif selon la revendication 12, **caractérisé en ce que** les moyens de fixation comportent une rondelle (19) portant ladite enveloppe et enfilée autour de la tige de l'organe de refroidissement (13).

14. Dispositif selon la revendication 13, **caractérisé en ce que** la rondelle (19) comporte une face située en vis-à-vis du cristal (11) sur laquelle est déposé un revêtement (191) en matériau réfléchissant.

15. Dispositif selon la revendication 14, **caractérisé en ce que** lesdits moyens de fixation comportent une ou plusieurs entretoises (17) annulaires positionnées autour de la surface latérale dudit cristal et supportant ladite enveloppe.

16. Dispositif selon la revendication 15, **caractérisé en ce que** chaque entretoise (17) comporte plusieurs lamelles (170) souples venant en appui contre la surface latérale dudit cristal (11).

**Patentansprüche**

1. Vorrichtung zur lokalisierten Kühlung, umfassend:

   - Eine Kühleinrichtung (13),
   - Einen Kristall (11) mit Fähigkeit zur Kühlung durch Absorption eines Nahinfrarot-Anregungslichtsignals,
   - Ein Beleuchtungssystem (14) zur Bereitstellung eines Anregungslichtsignals:
   - Wobei der Kristall (11) eine längliche Form um eine Längsachse zwischen einem proximalen Ende und einem distalen Ende aufweist und einen geschlossenen konstanten Außenquerschnitt und einen Mittelkanal, der ausgehend von seinem distalen Ende über wenigstens einen Abschnitt seiner Länge ausgebildet ist, aufweist,
   - Wobei die Kühleinrichtung (13) einen Stift aufweist, der mit einem ersten Ende in den Mittelkanal des Kristalls eingelassen ist und ein zweites, hervorstehendes Ende, das einen Kühlfinger (130) bildet, aufweist
   - Wobei das Beleuchtungssystem dazu ausgebildet ist, das Anregungslichtsignal in einer Ringform zu erzeugen.

2. Vorrichtung nach Anspruch 1, **dadurch gekennzeichnet, dass** der Kristall (11) einen konstanten ringförmigen Querschnitt zwischen seinem proximalen Ende und seinem distalen Ende aufweist.

3. Vorrichtung nach Anspruch 1, **dadurch gekennzeichnet, dass** das Beleuchtungssystem eine Lichtquelle, die dazu ausgebildet ist, ein erstes Lichtsignal abzugeben, und ein Modul (141) zur Formung des ersten Lichtsignals, das zwischen der Lichtquelle (140) und dem proximalen Ende des Kristalls (11) angeordnet ist und dazu ausgebildet ist, das Anregungssignal in der Ringform zu erzeugen, aufweist.

4. Vorrichtung nach Anspruch 3, **dadurch gekennzeichnet, dass** das Formungsmodul (141) eine erste Axicon-Linse (15a) und eine zweite Axicon-Linse (15b) aufweist.

5. Vorrichtung nach einem der Ansprüche 1 bis 4, **dadurch gekennzeichnet, dass** der Kristall (11) vom Typ Yb:YLF oder Tm:Yb:YLF ist.

6. Vorrichtung nach einem der Ansprüche 1 bis 5, **dadurch gekennzeichnet, dass** der Finger (130) der Kühleinrichtung (13) eine atraumatische Form aufweist.

7. Vorrichtung nach einem der Ansprüche 1 bis 6, **dadurch gekennzeichnet, dass** sie Mittel zur Wiederverwertung der Fluoreszenz aufweist, die bei der Anregung des Kristalls (11) erzeugt wird.

8. Vorrichtung nach Anspruch 7, **dadurch gekennzeichnet, dass** der Kristall (11) eine Seitenfläche aufweist, und dadurch, dass die Mittel zur Wiederverwertung der Fluoreszenz einen oder mehrere dichroitische Filter (16) aufweisen, die am Rand der Seitenfläche des Kristalls angeordnet sind, eine seitliche Hülle des Kristalls bildend, die von der Seitenfläche des Kristalls wärmeisoliert ist.

9. Vorrichtung nach Anspruch 8, **dadurch gekennzeichnet, dass** die Mittel zur Wiederverwertung der Fluoreszenz Fluorophorelemente (18) aufweisen.

10. Vorrichtung nach Anspruch 9, **dadurch gekennzeichnet, dass** die Hülle mindestens zwei dichroitische Filter (16a, 16b) aufweist, und dadurch, dass die Fluorophorelemente (18) zwischen den zwei dichroitischen Filtern angeordnet sind.

11. Vorrichtung nach Anspruch 9 oder 10, **dadurch gekennzeichnet, dass** die Hülle aus einem einstückigen Zylinder oder mehreren benachbarten Plättchen, die aneinander liegend zusammengefügt sind, gebildet ist.

12. Vorrichtung nach einem der Ansprüche 9 bis 11, **dadurch gekennzeichnet, dass** sie Mittel zur Befestigung der Hülle aufweist.

13. Vorrichtung nach Anspruch 12, **dadurch gekennzeichnet, dass** die Befestigungsmittel eine Scheibe (19) aufweisen, die die Hülle trägt und um den Stift der Kühleinrichtung (13) gesteckt ist.

14. Vorrichtung nach Anspruch 13, **dadurch gekennzeichnet, dass** die Scheibe (19) eine Seite aufweist, die sich gegenüber dem Kristall (11) befindet, auf der eine Beschichtung (191) aus reflektierendem Material aufgebracht ist.

15. Vorrichtung nach Anspruch 14, **dadurch gekennzeichnet, dass** die Befestigungsmittel einen oder mehrere ringförmige Abstandshalter (17) aufweisen, die um die Seitenfläche des Kristalls positioniert sind und die Hülle stützen.

16. Vorrichtung nach Anspruch 15, **dadurch gekennzeichnet, dass** jeder Abstandshalter (17) mehrere nachgiebige Lamellen (170) aufweist, die gegen die Seitenfläche des Kristalls (11) in Anlage kommen.

**Claims**

1. Device for cooling locally, comprising:

   - a cooling member (13),
   - a crystal (11) having the capacity to cool via absorption of a near-infrared exciting light signal,
   - an illuminating system (14) intended to deliver an exciting light signal, **characterized in that**:
   - said crystal (11) has an elongate shape about a longitudinal axis between a near end and a far end and has a closed constant outside cross section and contains a central channel formed, from its far end, over at least some of its length,

- said cooling member (13) comprises a rod embedded via a first end into said central channel of said crystal and comprises a protruding second end that forms a cooling finger (130),
- the illuminating system is configured to generate said exciting light signal with an annular shape.

2. Device according to Claim 1, **characterized in that** the crystal (11) has a constant cross section of annular shape between its near end in its far end.

3. Device according to Claim 1, **characterized in that** the illuminating system comprises a light source configured to emit a first light signal and a shaping module (141) for shaping said first light signal arranged between said light source (140) and the near end of said crystal (11) and configured to generate said exciting signal with the annular shape.

4. Device according to Claim 3, **characterized in that** the shaping module (141) comprises a first axicon lens (15a) and a second axicon lens (15b).

5. Device according to one of Claims 1 to 4, **characterized in that** the crystal (11) is of Yb:YLF or Tm:Yb:YLF.

6. Device according to one of Claims 1 to 5, **characterized in that** the finger (130) of the cooling member (13) has an atraumatic shape.

7. Device according to one of Claims 1 to 6, **characterized in that** it comprises means for recycling the fluorescence generated during the excitation of said crystal (11).

8. Device according to Claim 7, **characterized in that** said crystal (11) comprises a lateral surface and **in that** said means for recycling the fluorescence comprise one or more dichroic filters (16) that are arranged on the periphery of said lateral surface of the crystal, and that form a lateral covering of said crystal, said covering being thermally insulated from the lateral surface of the crystal.

9. Device according to Claim 8, **characterized in that** the means for recycling the fluorescence comprise fluorophore elements (18).

10. Device according to Claim 9, **characterized in that** said covering comprises at least two dichroic filters (16a, 16b) and **in that** fluorophore elements (18) are arranged between the two dichroic filters.

11. Device according to Claim 9 or 10, **characterized in that** said covering is composed of a single-piece cylinder or of a plurality of adjacent sheets that are assembled with one another in a continuous way.

12. Device according to one of Claims 9 to 11, **characterized in that** it comprises means for fastening said covering.

13. Device according to Claim 12, **characterized in that** the fastening means comprise a washer (19) bearing said covering and slipped around the rod of the cooling member (13).

14. Device according to Claim 13, **characterized in that** the washer (19) has a face located facing the crystal (11), on which face is deposited a coating (191) made of reflective material.

15. Device according to Claim 14, **characterized in that** said fastening means comprise one or more annular spacers (17) that are positioned around the lateral surface of said crystal and that hold said covering.

16. Device according to Claim 15, **characterized in that** each spacer (17) comprises a plurality of flexible strips (170) that bear against the lateral surface of said crystal (11).

**Fig. 1A**

**Fig. 1B**

**Fig. 2A**

**Fig. 2B**

A-A

*Fig. 3*

*Fig. 4*

*Fig. 5A*

*Fig. 5B*

*Fig. 5C*

**Fig. 6A**

**Fig. 6B**

**Fig. 6C**

C-C

**Fig. 6D**

**Fig. 6E**

**Fig. 6F**

## Fig. 7A

## Fig. 7B

D-D

## Fig. 8

## Fig. 9

| Fluorescence parasite – Evacuation ou conversion | Fluorescence utile - recyclage |

λf=989nm

Conversion

## Fig. 10A

16b

18

16a

11

F

F2

Fig. 10B

## Fig. 11A

## Fig. 11B

## Fig. 12A

## Fig. 12B

## Fig. 13A

## Fig. 13B

## RÉFÉRENCES CITÉES DANS LA DESCRIPTION

### Documents brevets cités dans la description

- US 2007005121 A1
- JP 2006015064 A
- US 5620571 A
- US 9362712 B1
- WO 2016102351 A1
- US 6041610 A
- WO 0042683 A1
- WO 2018051005 A1
- US 20170137684 A1